# EUROPEAN PATENT APPLICATION

(11) **EP 4 324 916 A1**
(43) Date of publication of application: **21.02.2024**
(21) Application number: 22788269.3
(22) Date of filing: 11.03.2022
(51) Int. Cl.: C12N 5/0783, C07K 14/705, C07K 14/47, A61K 35/17, A61P 35/00, A61P 31/00

(54) **METHOD FOR ACTIVATING AND PROLIFERATING CD8 T CELLS EXPOSED TO ANTIGEN, CD8 T CELLS, PREPARED THEREBY, HAVING ENHANCED ANTICANCER ACTIVITY, AND USE THEREOF**

(30) Priority: 12.04.2021 KR 20210047002; 17.09.2021 US 202117478445
(71) Applicant: Medgene Therapeutics, Inc., Silver Spring, MD 20902 (US)
(72) Inventor: SEONG, Rho Hyun, Seongnam-si, Gyeonggi-do 13589 (KR); NAH, Jin Woo, Seoul 08748 (KR)
(74) Representative: Dehns
(86) International application number: PCT/KR2022/003482
(87) International publication number: WO 2022/220412

(57) **Abstract**

The present invention relates to a method for activating a cell and a cell activated thereby and a use thereof, more particularly, to an *in vitro* method of enhancing and recovering of immune response of CD8 T cells in exhaustion and proliferating the CD8 T cells comprising the step of inducing overexpression of Klf4 protein in CD8 T cells isolated from a subject, a cell population containing the CD8 T cells or transduced CAR-CD8 T cells whose anticancer activity is enhanced by overexpressing Klf4 protein and use thereof.

## Description

### TECHNICAL FIELD

The present invention relates to a method of cell proliferation and activation, a cell activated thereby and a use thereof, and more particularly, to a method of activating and proliferating CD8 T cells continuously exposed to an antigen, and CD8 T cells with enhanced anticancer activity produced by the same and uses thereof.

### BACKGROUND OF THE INVENTION

Most CD8 T cells infiltrated the tumor tissue recognize specific antigens derived from cancer cells. The antigen (Ag)-activated CD8 T cells can eliminate cancer cells by secreting effector molecules such as granzyme B, interferon-gamma (IFN-γ), and perforin. Therefore, increasing the activity of the CD8 T cells specific for cancer antigens is regarded as one of the most efficient approaches to treat cancers. Check point inhibitors such as anti-PD-1 and anti-CTLA antibodies (Abs) work to control cancers in such a way, however, the efficiency is quite limited. It is recognized that controlling the CD8 T cell activity in cancer tissues is highly sophisticated because of the complex characteristics of cancer microenvironment.

In the cancer microenvironment, antigen-specific CD8 T cells are continuously exposed to cancer antigens unless cancer is completely removed. Chronic antigen stimulation makes CD8 T cells fall into unresponsive state which is currently described as 'exhaustion'. Exhausted CD8 T cells are not able to efficiently eliminate cancer cells because they cannot perform a normal immune response due to the weakening of active cytokine secretion and cell division functions. In particular, these exhausted CD8 T cells have a characteristic that they do not easily return to cells with normal functions even after time passes. Therefore, preventing CD8 T cells from being exhausted in the tumor tissue and reactivating the function of the exhausted cells is critical to control cancers.

Recent studies have shown that CD8 T cells from chronic virus infected tissues or tumor tissues are composed of 4 subsets of cell groups displaying stages of exhaustion; These include chronic progenitor cell subset (Progenitor exhausted cells 1, Progenitor exhausted cells 2), chronic effector cell subset (intermediate exhausted cells) and end-stage exhausted cell subset (terminal exhausted cells) . Among them, chronic effector cell subset has the highest effector function to recognize and remove cancer cells, but terminal exhausted cells are known to have lost a significant part of an effector function. Indeed, it is known that most of the CD8 T cells present in cancer tissues do not function properly since they are in the terminal state of exhaustion. Therefore, if a method for enhancing the generation and function of chronic effector cells from cancer-specific CD8 T cells is developed, it will be very useful to effectively control and treat cancer.

With respect to anticancer treatment using CD8 T cells, US Patent US8106092 discloses a method of treating secondary cancer by inducing necrosis of cancer cells and promoting generation of cancer-specific T cells such as CD8 T cells, comprising administrating ingenol-3-angelate locally and/or intratumorally to an individual with secondary cancer, and US Patent Publication No. US20190218515A discloses a method of producing activated T cells comprising treating T cells isolated from cancer patients with a dual specific antibody against CD123/CD3, a dual-specific antibody against CD19/CD3 or a dual-specific antibody against EpCAM/CD3.

On the other hand, T cell therapeutics are being developed up to the 3^{rd} generation so far. The first generation T cell therapeutics have low specificity for cancer cells because they are administered to patients by proliferating all T cells (bulk T cells) present in the blood or cancer tissue. Efficacy could not be expected, and the second-generation T cell therapeutics showed an improved therapeutic effect by isolating/mass-culturing only tumor antigen-specific T cells and administering them to cancer patients. The problem of this long and complicated process has been raised, and the third-generation T cell therapeutics have been developed by either 1) directly introducing a TCR gene that recognizes a specific cancer antigen into T cells, or 2) preparing a fusion protein by linking an antigen recognition site (scFv) of a monoclonal antibody that recognizes a specific antigen to a T cell activation domain and then introducing the fusion protein into T cells, and thereby increased antigen specificity and shortening the manufacturing period, and the therapeutic efficacy is also very good, close to 100% treatment in case of some leukemias and lymphomas. The second type of third-generation T cell therapy is characterized by being genetically engineered to express the so-called chimeric antigen receptor (hereinafter, abbreviated as 'CAR').

The leading companies based on the CAR-introduced T-cell technology are Novartis, Juno Therapeutics, and Kite Pharma of the United States, all of which have developed CAR-introduced T cells that target CD19, a B-cell-specific antigen. The CAR-T cell therapeutics developed by the companies showed a high therapeutic effect of 80 to 90% in resistant/recurrent acute lymphoblastic lymphomas (ALL) and non-Hodgkin's lymphoma (NHL), while positioning them as leaders in targeted immune cell therapies (Hartmann et al., EMBO Mol. Med. 9 (9): 1183-1197, 2017). Furthermore, one of the world's first chimeric antigen receptor T-cell (CAR-T) therapeutics, 'Cymriaju' (Indgredient Name: Tisagen Lexel) recently applied for approval by Novartis Korea, was approved as the No. 1 high-tech biopharmaceutical in accordance with ^{┌}Advanced Regenerative Bio Act_{┘} of Korea.

### DISCLOSURE

### TECHNICAL PROBLEM

However, the prior technologies also have limitations in that they do not take into account the phenomenon of CD8 T cells being exhausted in the body and do not aim to utilize these specialized cells or to overcome this condition.

The present invention is to solve various problems related to T cell exhaustion including the above problems in T cell immunotherapy. Thus the object of the present invention is to provide a method and composition capable of controlling cancer by regulating the exhaustion process of CD8 T cells *in vivo.* However, these problems are exemplary and do not limit the scope of the present invention.

### TECHNICAL SOLUTION

In an aspect of the present invention, there is provided an *in vitro* method of suppressing the exhaustion state of CD8 T cells comprising inducing overexpression of Klf4 protein in CD8 T cells selected from the group consisting of a) CD8 T cells, b) a cell population comprising the CD8 T cells, and c) transformed CAR-CD8 T cells prepared by transducing the CD8 T cells with a gene encoding a chimeric antigen receptor (CAR).

In another aspect of the present invention, there is provided an *in vitro* method of enhancing immune response of CD8 T cells comprising inducing overexpression of Klf4 protein in CD8 T cells selected from the group consisting of a) CD8 T cells, b) a cell population comprising the CD8 T cells, and c) transformed CAR-CD8 T cells prepared by transducing the CD8 T cells with a gene encoding a chimeric antigen receptor (CAR).

In another aspect of the present invention, there is provided an *in vitro* method of proliferating CD8 T cells isolated from a subject comprising inducing overexpression of Klf4 protein in CD8 T cells selected from the group consisting of a) CD8 T cells, b) a cell population comprising the CD8 T cells, and c) transduced CAR-CD8 T cells prepared by transducing the CD8 T cells with a gene encoding a chimeric antigen receptor (CAR).

In another aspect of the present invention, there is provided a pharmaceutical composition for treating cancer comprising CD8 T cells in which Klf4 is overexpressed or transformed CAR-CD8 T cells transduced to overexpress the Klf4 protein and a chimeric antigen receptor as an active ingredient.

In another aspect of the present invention, there is provided a transformed CD8 T cell transduced to overexpress Klf4 protein.

In another aspect of the present invention, there is provided a transformed CAR-CD8 T cell transduced to overexpress Klf4 protein and a chimeric antigen receptor (CAR).

In another aspect of the present invention, there is provided a composition comprising the transformed CD8 T cell or the transformed CAR-CD8 T cell.

In another aspect of the present invention, there is provided a method of treating a subject suffering from cancer comprising:
inducing overexpression of Klf4 protein in CD8 T cells isolated from a subject suffering from cancer or a cell population containing the CD8 T cells; and
administrating the CD8 T cells, or the cell population comprising the CD8 T cells whose overexpression of Klf4 protein is induced to the subject.

### EFFECTS OF THE INVENTION

As described above, the method according to an embodiment of the present invention can be usefully used in the development of more efficient anticancer cell therapeutics by preventing the exhaustion of immune cells.

### BRIEF DESCRIPTION OF DRAWINGS

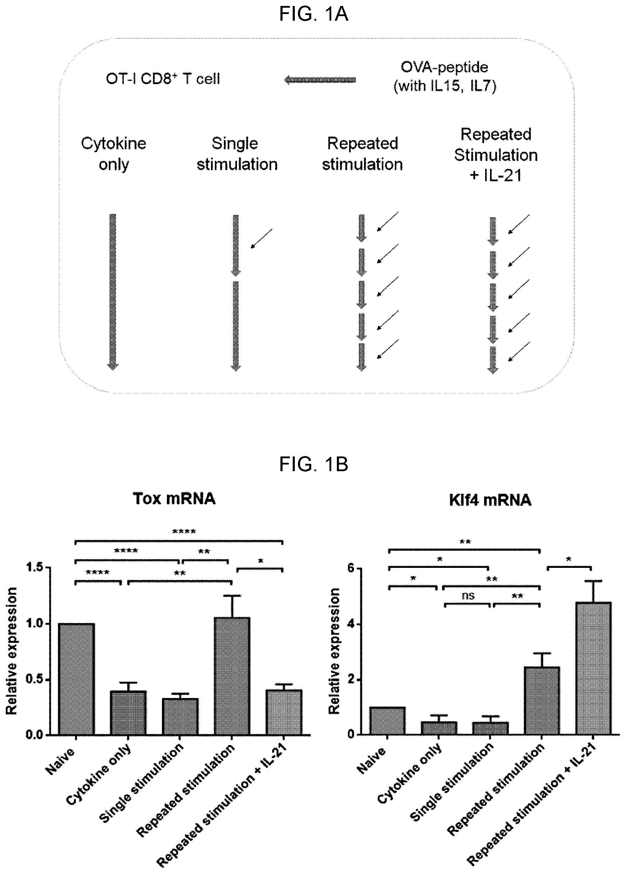

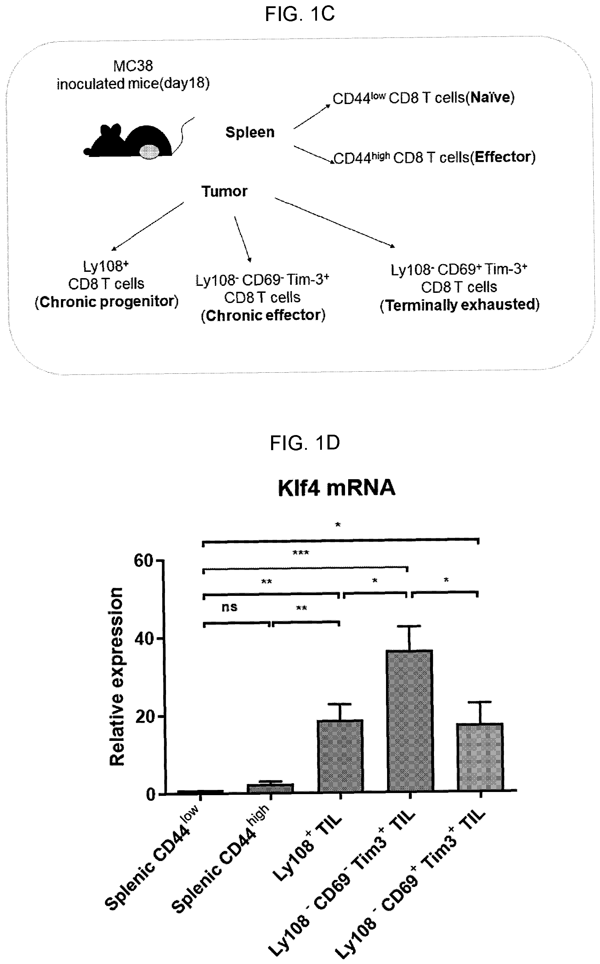

FIG. 1A is a schematic diagram schematically showing the design of a repeated stimulation test for antigen on CD8 T cells according to an embodiment of the present invention; FIG. 1B is a series of graphs showing the results of measuring the expression level at the mRNA level of Tox (left) and Klf4 (right), markers related to the exhaustion state of CD8 T cells showing experimental results performed according to the experimental design of FIG. 1A; FIG. 1c is a schematic diagram representing the marker phenotypes for various stages of CD8 T cell subsets from spleen and from tumor tissue induced by inoculating MC38 colon cancer cells into mice; and FIG. 1D is a graph showing the result of measuring expression level of Klf4 protein in the CD8 T cell subsets at the mRNA level.

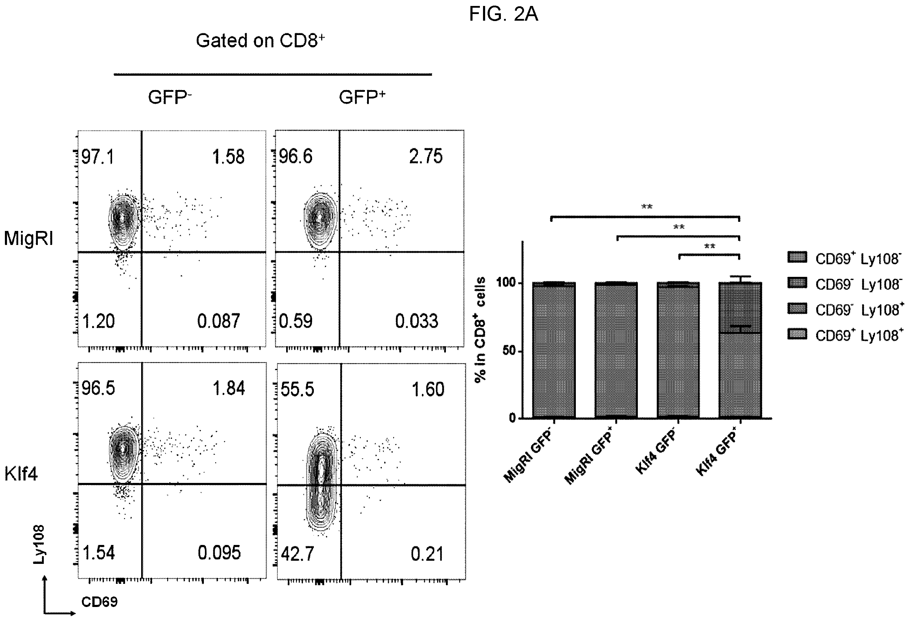

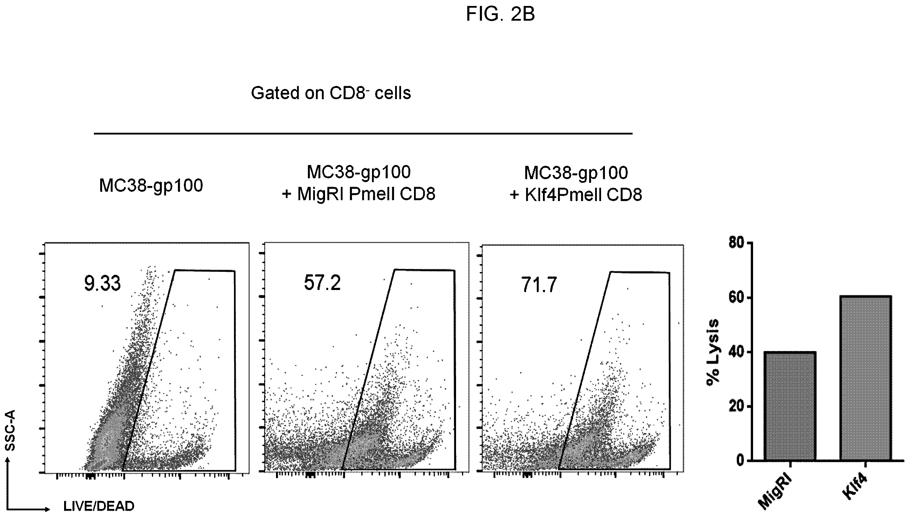

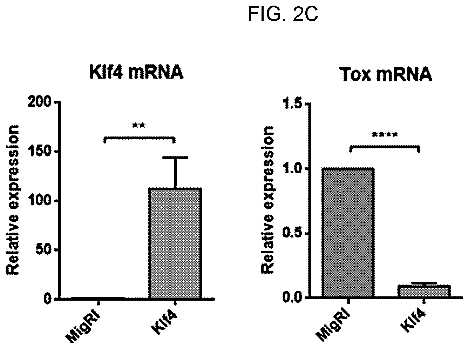

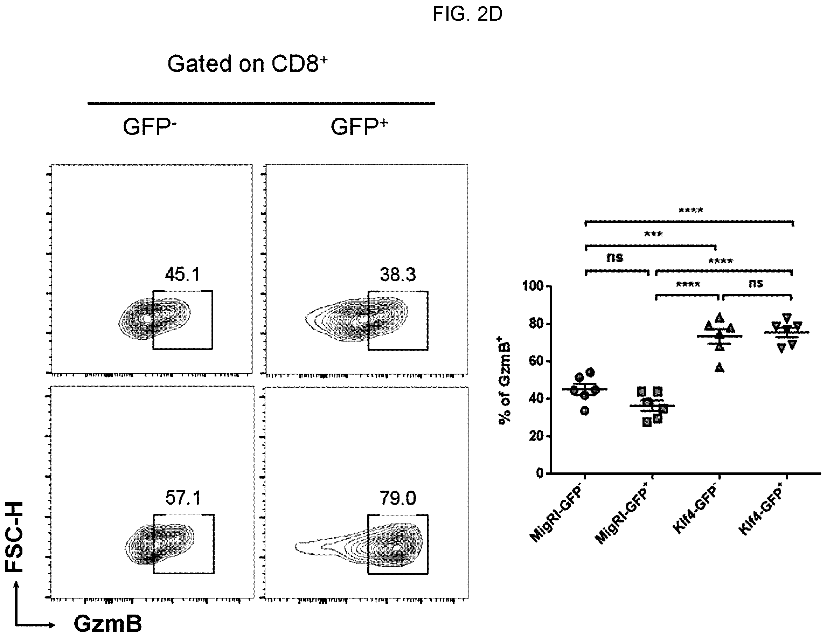

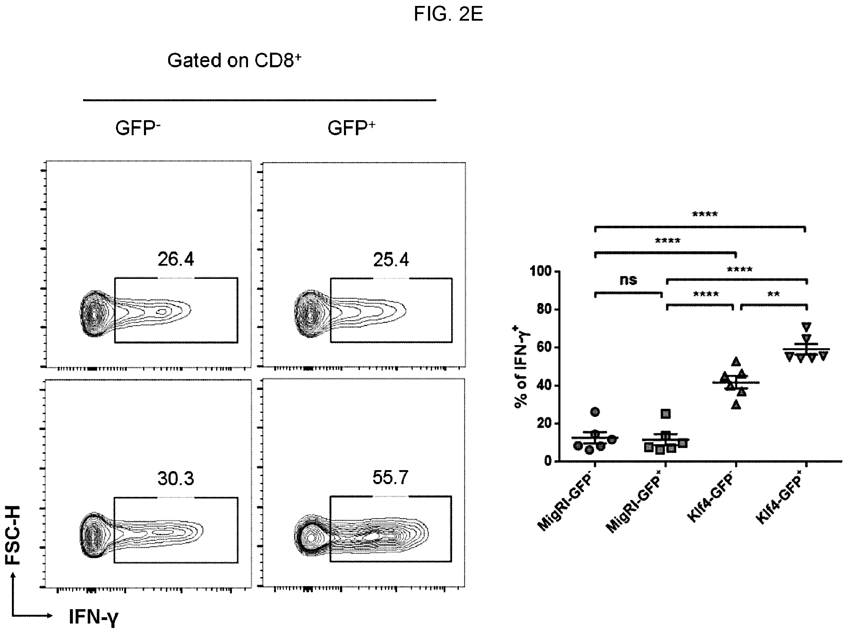

FIGs. 2A to 2E show the results of characterization of CD8 T cells transformed with a control retroviral vector (MigRI) or CD8 T cells transformed with a retroviral vector (Klf4) containing the Klf4 gene, according to an embodiment of the present invention. FIG. 2A shows a series of histograms (left) representing the result of FACS analyses on CD8 T cells transformed with a control retroviral vector (MigRI) or CD8 T cells transformed with a retroviral vector (Klf4) using markers specific to aforementioned subsets and a graph (right) representing the result of the FACS analyses; FIG. 2B shows a histogram (left) representing ratio of dead cancer cells when cultivating CD8 T cells transformed with control (MigRI) vector or the Klf4 gene with target cancer cells and a graph quantifying the FACS results; FIG. 2C is a series of graphs representing the results of measuring the gene expression level of Klf4 (left) and Tox (right) at the mRNA level in the control (MigRI) and CD8 T cells transformed with the Klf4 gene; FIG. 2D shows a series of histograms (left) representing FACS analyses showing the proportion of CD8 T cells expressing granzyme B (GzmB) in four groups of CD8 T cells (clockwise from top left, MigRI-GFP⁻, MigRI-GFP⁺, Klf4-GFP⁺, Klf4-GFP⁻) and a graph (right) quantifying the FAC analyses; and FIG. 2E shows a series of histograms (left) representing FACS analyses showing the proportion of CD8 T cells expressing interferon-γ (INF- γ), which has a key role in the function of CD8 T cells in four groups of CD8 T cells (clockwise from top left, MigRI-GFP⁻, MigRI-GFP⁺, Klf4-GFP⁺, Klf4-GFP⁻) and a graph (right) quantifying the FACS analyses.

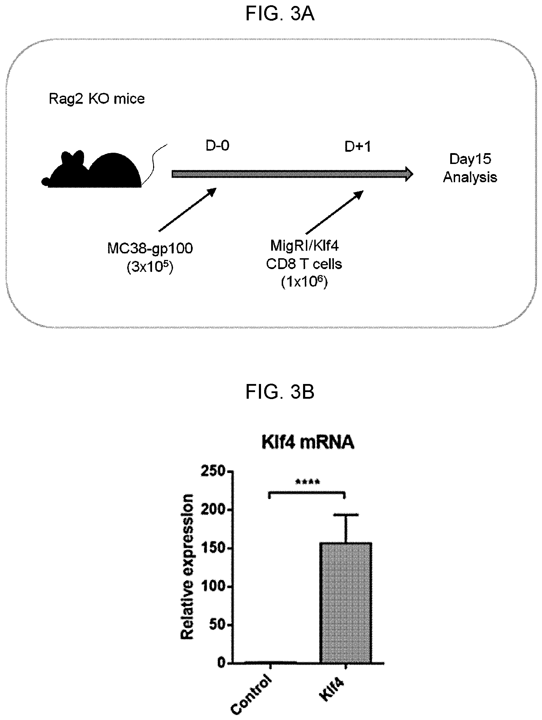

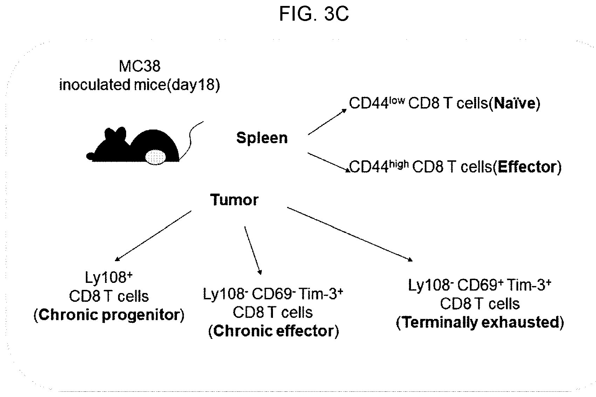

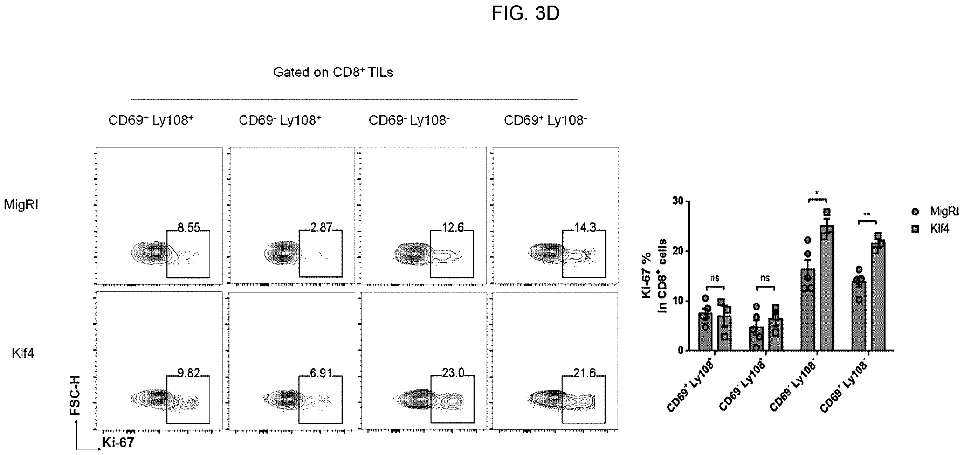

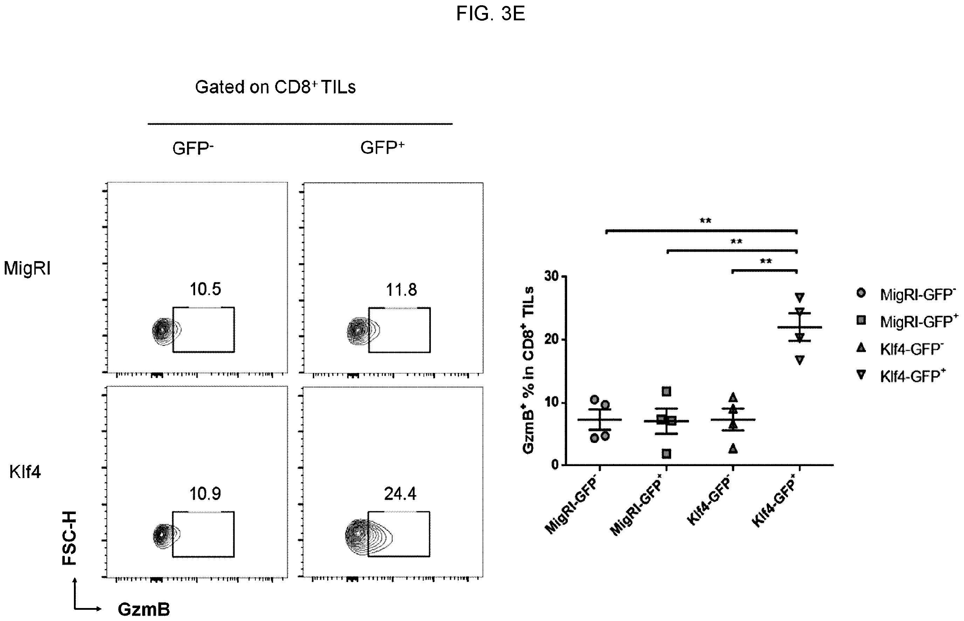

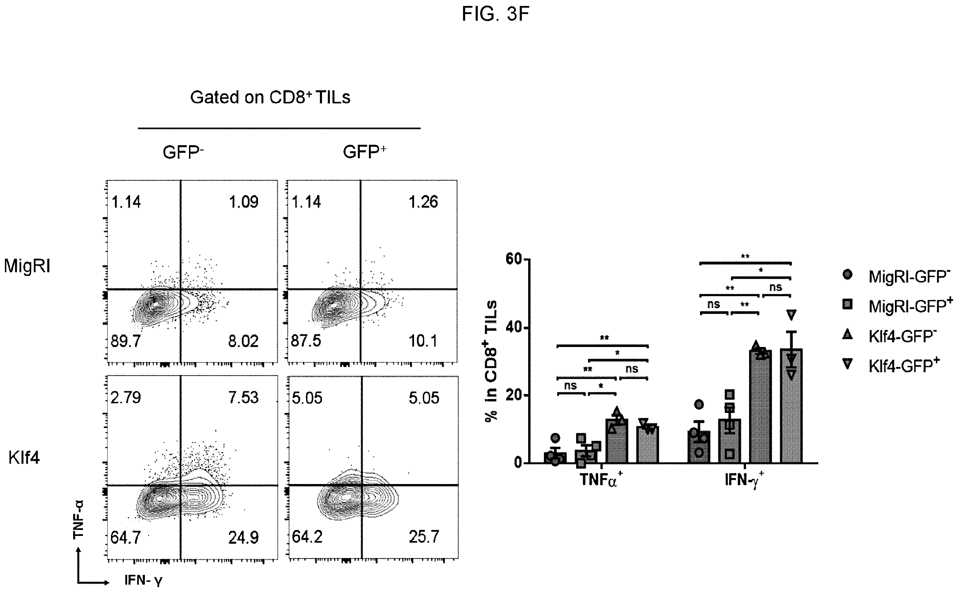

FIG. 3A is a schematic diagram schematically illustrating an administration schedule of an animal experiment using CD8 T cells transduced with Klf4 gene according to an embodiment of the present invention; FIG. 3B is a graph showing the result of measuring the expression level of the Klf4 gene at the level of mRNA in the control group (Control) and CD8 T cells transduced with Klf4 gene (Klf4); FIG. 3C shows a graph showing the change in the volume of the tumor tissue over time from excised from experimental animals administered with a control CD8 T cells (MigRI) or a CD8 T cell transformed with Klf4 gene (Klf4) according to an embodiment of the present invention, and a representative photograph of excised tumor tissues from the control group (MigRI) and experimental group (Klf4); FIG. 3D shows a series of FACS histograms representing the proportion of CD8 T cells expressing Ki-67 indicating the degree of cell division in the various CD8 T cell subsets isolated from the tumor tissues of the animals sacrificed after the animal experiment (left), and a graph quantifying the FACS results (right); FIG. 3E shows a series of two-dimensional FACS histograms showing the results of measuring the ratio of granzyme B-expressing cells among CD8 T cells isolated from tumor tissues of animals sacrificed after the animal experiment (left) and a graph quantifying the FACS results (right); and FIG. 3F represents a series of histograms showing the results of analyzing the expression levels of TNF-α and INF-γ in CD8 T cells isolated from tumor tissues of animals sacrificed after the animal experiment by FACS analysis (left) and a graph quantifying the FACS results (right).

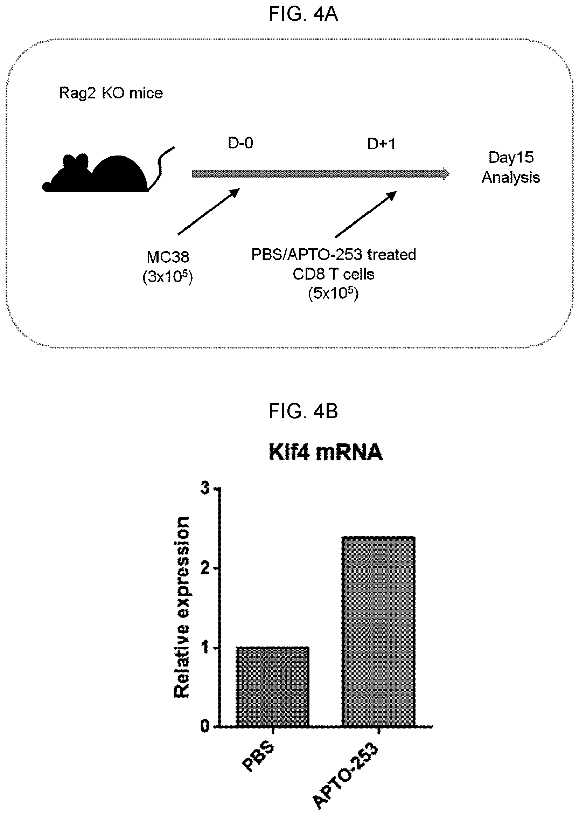

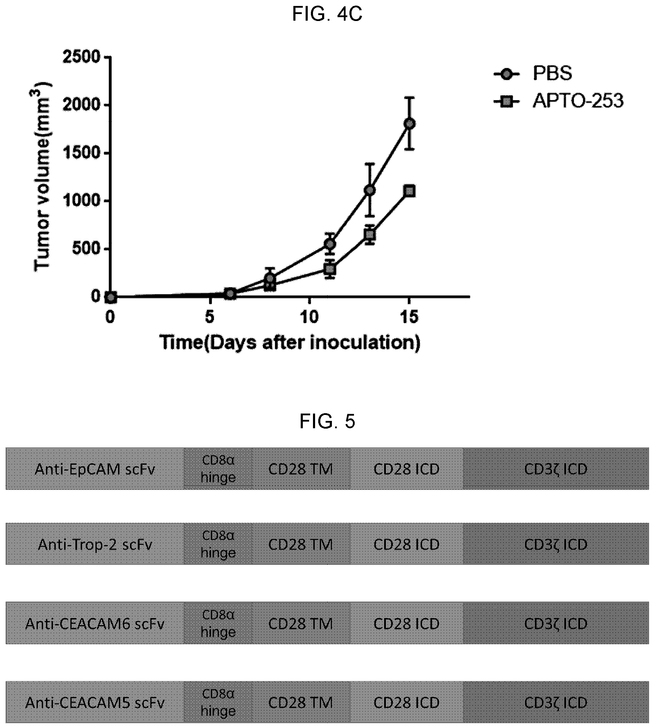

FIG. 4A is a schematic diagram schematically showing the administration schedule of an animal experiment using CD8 T cells treated with APTO-253 according to an embodiment of the present invention; FIG. 4B is a graph showing the results of measuring the expression level of the Klf4 gene at the mRNA level in the experimental animals administrated with CD8 T cells treated with PBS (control) or APTO-253; and FIG. 4C is a graph showing the change in the volume of the tumor tissues excised from the experimental animals administrated with CD8 T cells treated with PBS (control) or APTO-253 over time.
FIG. 5 is a schematic diagram showing various CAR constructs (EpCAM CAR, Trop-2 CAR, CEACAM6 CAR and CEACAM5 CAR) for the present invention.

### DETAILED DESCRIPTION OF THE INVENTION

In an aspect of the present invention, there is provided an *in vitro* method of suppressing the exhaustion state of CD8 T cells comprising inducing overexpression of Klf4 protein in CD8 T cells selected from the group consisting of a) CD8 T cells isolated from a subject, b) a cell population comprising the CD8 T cells, and c) CAR-CD8 T cells prepared by transducing the CD8 T cells with a gene encoding a chimeric antigen receptor (CAR).

In another aspect of the present invention, there is provided an *in vitro* method of enhancing immune response of CD8 T cells comprising inducing overexpression of Klf4 protein in CD8 T cells selected from the group consisting of a) CD8 T cells isolated from a subject, b) a cell population comprising the CD8 T cells, and c) CAR-CD8 T cells prepared by transducing the CD8 T cells with a gene encoding a chimeric antigen receptor (CAR).

In another aspect of the present invention, there is provided an *in vitro* method of proliferating CD8 T cells isolated from a subject comprising inducing overexpression of Klf4 protein in CD8 T cells selected from the group consisting of a) CD8 T cells isolated from a subject, b) a cell population comprising the CD8 T cells, and c) CAR-CD8 T cells prepared by transducing the CD8 T cells with a gene encoding a chimeric antigen receptor (CAR).

In the method, the overexpression of Klf4 protein is performed by transfecting the CD8 T cell with an expression vector containing a polynucleotide encoding the Klf4 protein, or introducing an mRNA expressing the Klf4 protein into the CD8 T cell, or treating the CD8 T cells with a Klf4 inducer. The Klf4 protein may comprise an amino acid sequence represented by SEQ ID Nos: 4 or 5. In addition, the polynucleotide encoding the Klf4 protein may comprise a nucleotide sequence represented by SEQ ID Nos: 1 or 6. However, the present invention is not limited thereto. Any other Klf4 proteins or polynucleotides encoding the same may be used. Preferably, Klf4 protein or polynucleotides encoding the same derived from mammals, particularly from primates such as chimpanzees, gorillas, orangutans, gibbons, cynomolgus monkeys, and rhesus monkeys may be used.

In the method, the expression vector may be a viral vector or a non-viral vector, and the viral vector may be an adeno-associated virus (AAV) vector, an adenovirus vector, an alphavirus vector, a herpes simplex virus vector, or a vaccinia virus vector. , Sendaivirus vector, flavivirus vector, radovirus vector, retroviral vector, herpesvirus vector, poxvirus vector or lentiviral vector. In the method, the non-viral vector may be a DNA vector, nanoparticles, cationic polymer, exosome, extracellular vesicle or liposome, and the DNA vector may be a plasmid vector, a cosmid vector, a phagemid vector, or an artificial human chromosome.

The expression vector may include an appropriate regulatory sequence linked to a polynucleotide encoding the Klf4 protein so that the Klf4 protein can be overexpressed in the CD8 T cell, wherein the construction sequence is operably linked to the polynucleotide. In this case, it may be referred to as a "gene construct" by integrating the regulatory sequence and a polynucleotide encoding the Klf4 protein operably linked thereto. The gene construct may include appropriate restriction enzyme recognition sites at both ends for cloning in an expression vector. As used herein, the term "operably linked to" refers to a nucleic acid sequence of interest (e.g., in an *in vitro* transcription/translation system or in a host cell) is linked to a regulatory sequence in such a way that its expression can be achieved. The term "regulatory sequence" includes promoters, enhancers and other regulatory elements (e.g., polyadenylation signals). Regulatory sequences include constitutive elements directing that a target nucleic acid can be constitutively expressed in various host cells, inducible elements directing that a target nucleic acid can be expressed only in specific tissues or cells (e.g., tissue-specific regulatory sequences). It can be understood by those skilled in the art that the design of the expression vector may vary depending on factors such as the selection of the host cell to be transformed and the level of desired protein expression. The regulatory sequences enabling expression in eukaryotic and prokaryotic cells are well known to those skilled in the art. As described above, they usually contain regulatory sequences responsible for initiation of transcription and, optionally, poly-A signals responsible for termination and stabilization of the transcript. Additional regulatory sequences may include, in addition to transcriptional regulators, translation enhancers and/or natively-combined or heterologous promoter regions. Possible regulatory sequences enabling expression in, for example, mammalian host cells are the CMV-HSV thymidine kinase promoter, SV40 promoter, RSV-promoter (Rous sarcoma virus), human kidney element 1α-promoter, glucocorticoid-inducible MMTV (mouse mammary tumor virus)-promoters, metallothionein-inducible promoter or tetracycline-inducible promoters, or enhancers such as CMV enhancer or SV40 enhancer. For expression in neurons, it is contemplated that neurofilament-promoter, PGDF-promoter, NSE-promoter, PrP-promoter or thy-1-promoter may be used. Such promoters are known in the art and are described in documents (Charron et al., J. Biol. Chem. 270: 25739-25745, 1995, etc.). For expression in prokaryotic cells, a number of promoters have been disclosed, including the lac-promoter, the tac-promoter or the trp promoter. In addition to factors capable of initiating transcription, the regulatory sequences include transcription termination signals such as SV40-poly-A site or TK-poly-A site downstream of the polynucleotide according to an embodiment of the present invention. In this document, suitable expression vectors are known in the art, such as Okayama-Berg cDNA expression vectors pcDV1 (Parmacia), pRc/CMV, pcDNA1, pcDNA3 (Invitrogen), pSPORT1 (GIBCO BRL), pX (Pagano et al., Science 255: 1144-1147, 1992), yeast two-hybrid vectors such as pEG202 and dpJG4-5 (Gyuris et al., Cell 75: 791-803, 1995) or prokaryotic expression vectors such as lambda gt11 or pGEX (Amersham-Pharmacia). In addition to the nucleic acid molecules of the present invention, the vector may further comprise a polynucleotide encoding a secretion signal. The secretion signals are well known to those skilled in the art. And, depending on the expression system used, a leader sequence capable of guiding the translated protein to the cell compartment such as nuclei is combined with the coding sequence of the polynucleotide according to an embodiment of the present invention, the leader sequence is preferably a leader sequence capable of secreting translated proteins around the cytoplasm or outside the cell.

In addition, the expression vector used in the present invention may be prepared by, for example, standard recombinant DNA technology, and the standard recombinant DNA technology includes, for example, ligation of blunt and adhesive ends, and treating with restriction enzymes for providing appropriate ends, removal of a phosphate group by alkaline phosphatase treatment to prevent improper bond, and enzymatic ligation by T4 DNA ligase, and the like. The vector of the present invention can be prepared by recombination of a DNA encoding a signal peptide obtained by chemical synthesis or genetic recombination technology, or a DNA encoding Klf4 protein according to an embodiment of the present invention, into a vector containing an appropriate regulatory sequence. A vector including the regulatory sequence can be purchased or prepared commercially.

In addition, in the method, the gene construct may further include a polynucleotide encoding one or more immune-stimulating peptides. In this case, the immune-stimulating peptide is in a form linked to a separate regulatory sequence, that is, in a bicistron form. It is included in the expression vector or linked to one regulatory sequence, but an internal ribosome entry site (IRES) may be inserted between the polynucleotides encoding the two proteins, transcribed into a single mRNA, and then translated into each protein. The immune-stimulating peptide may be CD28, ICOS (inducible costimulator), CTLA4 (cytotoxic T lymphocyte associated protein 4), PD1 (programmed cell death protein 1), BTLA (B and T lymphocyte associated protein), DR3 (death receptor 3), 4-1BB, CD2, CD40, CD40L, CD30, CD27, signaling lymphocyte activation molecule (SLAM), 2B4 (CD244), NKG2D (natural-killer group 2, member D)/DAP12 (DNAX-activating protein 12), TIM1 (T-cell immunoglobulin and mucin domain containing protein 1), TIM2, TIM3, TIGIT, CD226, CD160, LAG3 (lymphocyte activation gene 3), B7-1, B7-H1, GITR (glucocorticoid-induced TNFR family related protein), Flt3 ligand (fms-like tyrosine kinase 3 ligand), flagellin, herpesvirus entry mediator (HVEM), or the cytoplasmic domain of OX40L [ligand for CD134(OX40), CD252], or a linkage of two or more thereof.

In the method, the Klf4 inducer may be APTO-253 { 2-(5-fluoro-2-methyl-1H-indol-3-yl)-1H-imidazo[4,5-f] [1,10]phenanthroline}.

In another aspect of the present invention, there is provided a transformed CD8 T cell transduced to overexpress a heterologous Klf4 protein.

The transformed CD8 T cells can be prepared by transducing CD8 T cells isolated from a subject in need of treatment or a cell population comprising the same with an expression vector containing a polynucleotide encoding a Klf4 protein. The expression vector may be a viral vector or a non-viral vector, and the viral vector may be an adeno-associated virus (AAV) vector, an adenovirus vector, an alphavirus vector, a herpes simplex virus vector, a vaccinia virus vector, or a Sendai virus vector. , a flavivirus vector, a radovirus vector, a retroviral vector, a herpesvirus vector, a poxvirus vector or a lentiviral vector. The non-viral vector may be an mRNA, a DNA vector, nanoparticles, cationic polymer, exosome, extracellular vesicle, or a liposome. In the tranformed CD8 T cell, the mRNA may be used alone or in combination with the non-viral vector other than the mRNA encoding the Klf4 protein. In addition, the DNA vector may be a plasmid vector, a cosmid vector, a phagemid vector, or an artificial human chromosome.

In another aspect of the present invention, there is provided a transformed CAR-CD8 T cell transduced to overexpress a heterologous Klf4 protein and a chimeric antigen receptor (CAR).

The transformed CAR-CD8 T cell may be prepared by transfecting CD8 T cells with a gene construct comprising a polynucleotide encoding the heterologous Klf4 protein and a gene construct comprising a polynucleotide encoding the CAR, preferably by transfecting CD8 T cells isolated from a subject in need of treatment or a cell population comprising the CD8 T cells with a gene construct comprising a polynucleotide encoding the Klf4 protein and a gene construct comprising a polynucleotide encoding the CAR. The both gene constructs may be co-transfected after being cloned into individual vectors or transfected with only a single vector comprising both the gene constructs. In this case, each gene may be operably linked to a separate regulatory site, such as a promoter and/or enhancer, and expressed as individual mRNAs, or linked to an IRES under a single transcriptional regulator, expressed as a single mRNA, and then translation and post-translation modification process may be performed separately. Various techniques such as vectors and promoters for the transduction of foreign genes are as described above.

As used herein, the term "chimeric antigen receptor (CAR)" refers to a fusion protein composed of a single chain-based antibody mimetic such as scFv or sdAb as an antigen recognition site, a transmembrane domain, a costimulatory domain, an intracellular signaling domain. It is well known that when a gene encoding the CAR is transduced into immune cells such as T cells, cancer cell-specific antigens are recognized to enhance the anticancer activity of these immune cells against cancer cells.

In the transformed CAR-CD8 T cell, the CAR may be a fusion protein comprising a single chain-based antibody mimetic, a transmembrane domain, a costimulatory factor and a cytoplasmic signaling domain. The single chain-based antibody mimetic may bind to a cancer antigen or an antigen derived from a pathogen specifically. The cancer antigen may be EpCAM (epithelial cell adhesion molecule), Trop-2 (trophoblast cell surface antigen 2), CEACAM5 (CEA cell adhesion molecule 5), CEACAM6 (CEA cell adhesion molecule 6), carcinoembryonic antigen (CEA), prostate-specific antigen prostatic acid phosphatase (PAP), prostate-specific membrane antigen (PSMA), Her2/neu, MUC-1, BCR/ABL, alpha-fetoprotein (AFP), an antigen derived from Epstein-Barr virus such as LMP2a, an antigen derived from human hepatitis B virus (HBV), human hepatitis C virus, Proteinase 3, WT-1, G250, melanoma antigen gene (MAGE), B melanoma antigen (BAGE), G melanoma antigen, NY-ESO-1, tyrosinase, tyrosinase-related protein-1 (TRP-1), TRP-2, gp100, MART-1, Ig Idiotype, CDK4, caspase-8, β-catenin, BCR/ABL, human papilloma virus antigen (HPV E6/E7), HHV-8, 5T4, p53, CA-125, CA-72-4, CA-15-3, or CA-19-9. The antigen derived from a pathogen may be an antigen derived from a pathogenic microorganism, a virus or a parasite. The pathogenic microorganism may be a pathogenic bacterium or a pathogenic fungus. The pathogenic bacterium may be *Bordetella pertussis*, tetanus, diphtheria, *Helicobacter pylori, Pneumococcus* sp., *Mycobacterium tuberoculosis*, *Cholera* sp., *Staphylococcus* sp., *Shigella* sp., *Borrelia* sp. or *Salmonella* sp. The pathogenic fungus may be *Candida* sp., *Trichophyton* sp., *Aspergillus* sp., *Fonsecaea* sp., *Epidermophyton* sp., *Piedraia* sp., *Malassezia* sp., *Pseudallescheria* sp., *Basidiobolus* sp., *Conidiobolus* sp., *Rhinosporidium* sp., *Paracoccidioides* sp., *Cryptococcus* sp., *Blastomyces* sp., *Sporothrix* sp., *Mucor* sp., *Absidia* sp., *Rhizopus* sp., *Pneumocystis* sp., *Wangiella* sp., *Phialophora* sp., or *Schizophyllum* sp. In addition, the virus may be influenza virus, human papilloma virus (HPV), vesicular stomatitis virus, cytomegalovirus (CMV), hepatitis A virus (HAV), hepatitis B virus (HBV), hepatitis C virus (HCV), hepatitis D virus (HDV), hepatitis G virus (HGV), respiratory synctytial virus (RSV), herpes simplex virus (HSV), or human immunodeficiency virus (HIV).

As used in this document, "antibody mimetic" means a single chain peptide derived from antigen-binding fragment of an antibody or a peptide having a similar function as an antibody, an antigen-binding activity, but is not derived from the antibody rather derived from a non-immunoglobulin scaffold protein consists of a single chain unlike conventional full-length antibodies in which two heavy chains and two light chains form a heterotetrameric quaternary structure to exert their function. Specifically, such an antibody mimetic includes an antibody-based single-chain antibody fragment, scFv (Glockshuber et al., Biochem. 29(6): 1362-1367, 1990), which is a single-chain antibody fragment prepared by connecting the V_{H} and V_{L} of an antibody with a linker, sdAb (single domain antibody), an antibody fragment composed of a single variable region fragment of an antibody, and an antigen-binding fragment (V_{H}H, V_{NAR}, etc.) of an antibody derived from Camelidae or cartilaginous fish, an antibody composed of only a heavy chain without a light chain, Affibody derived from the Z domain of protein A (Nygren, P. A., FEBS J. 275(11): 2668-2676, 2008), Gamma-B crystallin or Affilin derived from Ubiquitin (Ebersbach et al., J. Mol. Biol. 372(1): 172-185, 2007), Affimer derived from Cystatin (Johnson et al., Anal. Chem. 84(15): 6553-6560, 2012), Affitin derived from Sac7d (Krehenbrink et al., J. Mol. Biol. 383 (5): 1058-1068, 2008), Alphabody derived from triple helix coiled coil protein (Desmet et al., Nat. Commun. 23(12): 1556-1561, 2005), DARPin derived from Ankyrin repeat motif (Stumpp et al., Drug Discov. Today. 13(15-16): 695-701, 2008), derived from SH3 domain of Fyn protein of Fynomer (Grabulovski et al., J. Biol. Chem. 282(5): 3196-3204, 2007), Kunitz domain peptide derived from the Kunitz domain of various protein inhibitors (Nixon and Wood, Curr. Opin. Drug Discov. Dev. 9(2) : 261-268, 2006), monobody derived from the 10^{th} type 3 domain of fibronectin (Koide and Koide, Methods Mol. Biol. 352: 95-109, 2007), nanoCLAMP derived from the carbohydrate binding module 32-2 (Suderman et al., Protein Exp. Purif. 134: 114-124, 2017), hagfish-derived variable lymphocyte receptor (VLR) (Boehm et al., Ann. Rev. Immunol. 30: 203-220, 2012), and repebodies engineered in order to enhancer antigen affinity based on the VLR (Lee et al., Proc. Natl. Acad. Sci. USA, 109: 3299-3304, 2012).

The term "scFv" used herein means an abbreviation of "single chain variable fragment" and is not an actual antibody fragment, and a type of fusion protein in which a heavy chain variable region (V_{H}) and a light chain variable region (V_{L}) of an antibody are linked via a linker peptide with about 25 a.a. length. Tt is known to have antigen-binding activity even though it is not a native antibody fragment (Glockshuber et al., Biochem. 29(6): 1362-1367, 1990).

In the transformed CAR-CD8 T cell, the transmebtrane domain may be a transmembrane domain derived from 4-1BB/CD137, activated NK cell receptor, immunoglobulin protein, B7-H3, BAFFR, BLAME(SLAMF8), BTLA, CD100(SEMA4D), CD103, CD160 (BY55), CD18, CD19, CD19a, CD2, CD247, CD27, CD276 (B7-H3), CD28, CD29, CD3 delta, CD3 epsilon, CD3 gamma, CD3 zeta, CD30, CD4, CD40, CD49a, CD49D, CD49f, CD69, CD7, CD84, CD8, CD8 alpha, CD8 beta, CD96 (Tactile), CDl la, CDl lb, CDl lc, CDl ld, CDS, CEACAM1, CRT AM, cytokine receptor, DAP-10, DNAM1(CD226), Fc gamma receptor, GADS, GITR, HVEM (LIGHTR), IA4, ICAM-1, ICAM-1, Ig alpha (CD79a), IL-2R beta, IL-2R gamma, IL-7R alpha, inducible T-cell costimulatory (ICOS), integrin, ITGA4, ITGA4, ITGA6, ITGAD, ITGAE, ITGAL, ITGAM, ITGAX, ITGB2, ITGB7, ITGBl, KIRDS2, LAT, LFA-1, LFA-1, a ligand binding to CD83, LIGHT, LTBR, Ly9 (CD229), lymphocyte function-associated antigen-1 (LFA-1; CDl-la/CD18), MHC type 1 molecule, NKG2C, NKG2D, NKp30, NKp44, NKp46, NKp80 (KLRF1), OX-40, PAG/Cbp, programmed cell death-1 (PD-1), PSGL1, SELPLG (CD162), signaling lymphocytic activating molecule (SLAM), SLAMF1 (CD150 or IPO-3), SLAMF4 (CD244; 2B4), SLAMF6 (NTB-A; Lyl08), SLAMF7, SLP-76, TNF receptor protein, TNFR2, TNFSF14, toll ligand receptor, TRANCE/RANKL, VLA1, or VLA-6.

As used herein, the term "costimulatory domain" refers to the cytoplasmic domain responsible for the T cell costimulatory function of a costimulatory factor, which is an immune-related protein that assists in T/NK activation.

In the transformed CAR-CD8 T cell, the costimulatory domain may be a cytoplasmic domain or a conjugate of at least two or more among selected from the group consisting of CD28, ICOS (inducible costimulator), CTLA4 (cytotoxic T lymphocyte associated protein 4), PD1 (programmed cell death protein 1), BTLA(B and T lymphocyte associated protein), DR3(death receptor 3), 4-1BB, CD2, CD40, CD30, CD27, SLAM(signaling lymphocyte activation molecule), 2B4(CD244), NKG2D (natural-killer group 2, member D)/DAP12 (DNAX-activating protein 12), TIM1 (T-Cell immunoglobulin and mucin domain containing protein 1), TIM2, TIM3, TIGIT, CD226, CD160, LAG3 (lymphocyte activation gene 3), B7-1, B7-H1, GITR (glucocorticoid-induced TNFR family related protein), HVEM(herpesvirus entry mediator) and OX40L (CD252).

As used herein, the term "cytoplasmic signaling domain" refers to a cytoplasmic domain responsible for transducing an intracellular signal that mediates an immune response when a ligand is bound to a receptor in an immune cell.

In the transformed CAR-CD8 T cell, the cytoplasmic signaling domain may be one or more cytoplasmic domains selected from the group consisting of CD3ζ, CD28, CD27, OX40/CD134, 4-1BB/CD137, FcεRIγ, ICOS/CD278, IL-2Rβ/CD122, IL-2Rα/CD132, DAP10, DAP12, and CD40.

According to one embodiment of the present invention, the transgenic CAR-CD8 T cell may suppress exhaustion and increase recognition of cancer-antigens and immune responses to recognized cancer-antigens, thereby increasing additive or synergistic effects in anticancer treatment.

In another aspect of the present invention, there is provided a composition comprising the CD8 T cell or the CAR-CD8 T cell.

In the composition, the CD8 T cells overexpressing Klf4 protein may be autologous CD8 T cells isolated from a subject in need of treatment or heterologous CD8 T cells isolated from other subject, but preferably autologous CD8 T cells. The CD8 T cells overexpressing Klf4 protein may be prepared by transducing the CD8 T cells with a polynucleotide encoding Klf4 protein and/or treating the CD8 T cells with a Klf4 inducer.

The description of the transducing procedure and the Klf4 inducer is the same as described above.

The composition may be used to treat a disease requiring an innate immune response, and the disease requiring an innate immune response may be cancer, a bacterial infection, a fungal infection, a viral infection, or a parasitic infection.

The composition may further include a pharmaceutically acceptable adjuvant, excipient or diluent in addition to the carrier.

As used herein, the term "pharmaceutically acceptable" refers to that is physiologically acceptable and does not normally cause gastrointestinal disorders, allergic reactions such as dizziness or similar reactions when administered to humans. Examples of such carriers, excipients and diluents include lactose, dextrose, sucrose, sorbitol, mannitol, xylitol, erythritol, maltitol, starch, gum acacia, alginate, gelatin, calcium phosphate, calcium silicate, cellulose, methyl cellulose, polyvinylpyrrolidone, water, methylhydroxybenzoate, propylhydroxybenzoate, talc, magnesium stearate and mineral oil. In addition, fillers, anti-agglomeration agents, lubricants, wetting agents, fragrances, emulsifiers and preservatives may be further included.

In addition, the pharmaceutical composition according to an embodiment of the present invention can be formulated using methods known in the art to enable rapid, sustained or delayed release of the active ingredient when administered to a mammal. Formulations include powders, granules, tablets, emulsions, syrups, aerosols, soft or hard gelatin capsules, sterile injectable solutions, and sterile powder forms.

The composition according to an embodiment of the present invention may be administered by various routes, and may be administered by general systemic administration or local administration, for example, subcutaneous injection, intrasynovial injection, intraperitoneal injection, intramuscular injection, or intravenous injection. However, it is not limited thereto.

The composition according to an embodiment of the present invention may be formulated in a suitable form together with a commonly used pharmaceutically acceptable carrier. Pharmaceutically acceptable carriers include, for example, carriers for parenteral administration such as water, suitable oils, saline, aqueous glucose and glycol, and may further include stabilizers and preservatives. Suitable stabilizers include antioxidants such as sodium bisulfite, sodium sulfite or ascorbic acid. Suitable preservatives include benzalkonium chloride, methyl- or propyl-paraben and chlorobutanol. In addition, the composition according to the present invention may contain a suspending agent, a solubilizing agent, a stabilizer, an isotonic agent, a preservative, an adsorption inhibitor, a surfactant, a diluent, an excipient, a pH adjuster, an analgesic agent, a buffer, and antioxidants and the like may be included as appropriate. Pharmaceutically acceptable carriers and agents suitable for the present invention, including those exemplified above, are described in detail in Remington's Pharmaceutical Sciences, 15th Edition, 1975, Mack Publishing Company, Easton, Pennsylvania 18042 (Chapter 87: Blaug, Seymour), a formulation generally known in all pharmaceutical chemistries.

In addition, the composition of the present invention is administered in a therapeutically effective amount.

As used herein, the term "therapeutically effective amount" means an amount sufficient to treat a disease with a reasonable benefit/risk ratio applicable to medical treatment, and effective dose level includes the subject type and severity, age, sex, drug activity, sensitivity to drugs, administration time, administration route and excretion rate, duration of treatment, factors including concomitant drugs, and other factors well known in the medical field. The pharmaceutical composition of the present invention may be administered at a dose of 0.1 mg/kg to 1 g/kg, and more preferably at a dose of 1 mg/kg to 500 mg/kg. Meanwhile, the dosage may be appropriately adjusted according to the patient's age, sex, and condition.

In another aspect of the present invention, there is provided a method of treating a subject suffering from cancer comprising:
inducing overexpression of Klf4 protein in CD8 T cells isolated from a subject suffering from cancer or a cell population comprising the CD8 T cells; and
administrating the CD8 T cells, or the cell population comprising the CD8 T cells in which Klf4 protein is overexpressed to the subject.

In the method, the CD8 T cells or the cell population containing the CD8 T cells may be further transduced with a polynucleotide encoding a chimeric antigen receptor targeting a cancer antigen.

### BEST MODE FOR THE INVENTION

Hereinafter, the present invention will be described in more detail by following examples and experimental examples. It will be apparent to those skilled in the art that the present invention is not limited to the disclosed examples, but may be embodied in many different forms and the examples are provided in order to complete the disclosure of the present invention and to fully convey the scope of the invention to those skilled in the art.

### Examples

### Conventional Procedures

### Construction of retrovirus for Klf4 gene transduction

Platinum E cell line (platE, CELL BIOLABS, USA) was cultivated with DMEM medium (WELGENE) supplemented with 10% FBS (Gibco, USA), antibiotics streptomycin & penicillin (100 U/ml, WELGENE), and blastomycin (10 ug/ml, Gibco), and puromycin (1 ug/ml, Gibco) with care not to overgrow in consideration of cell confluency in a 5% CO₂ cell incubator at 37°C. When the cell confluency reached about 90%, the medium was removed, and then 5 ml of PBS (Sigma) was added to wash and then removed, and this washing process was repeated once more. Then, 2-3 ml of trypsin-EDTA solution (WELGENE) was added dropwise, and incubated for 3 minutes in a cell incubator at 37°C. Thereafter, the separated cells with 10 ml of DMEM medium were well pipetted, transferred to a 15 ml tube, and centrifuged for 5 minutes at 4°C at 1,500 rpm. The supernatant was removed, and the pellet was resuspended in DMEM medium (antibiotic-free medium) supplemented with only 10% FBS, and then cells were counted. 2.5×10⁶ platE cells in a 60 mm culture dish were resuspended in 3 ml of antibiotic-free DMEM medium, uniformly seeded, and cultured in a 37°C 5% CO₂ cell incubator for 6-8 hours. MigRI-Klf4 vector was prepared by inserting the CDS sequence (SEQ ID NO: 1) of the mouse klf4 gene into MigRI vector (Addgene, USA). At this time, cloning of the mouse klf4 gene was performed through PCR amplification using a primer set (SEQ ID NOs: 2 and 3) to which restriction enzymes (*Bgl*II and *Eco*RI) recognition sites for cloning into the MigRI vector were added. ACC sequence was inserted as a Kozak sequence between the recognition site and the initiation codon (ATG). Then, 312.5 pl of 2X HBS was dispensed into one 1.5 ml centrifuge tube, and 38.75 pl of 2 M CaCl₂ and 10 µg of DNA vector were added to the other centrifuge tube, and the remainder was distilled using tertiary distilled water. Then, the centrifuge tube in which 2X HBS is dispensed is vortexed at low intensity, and the solution in the centrifuge tube in which the DNA vector is dispensed is transferred to the centrifuge tube in which 2X HBS is dispensed in a dropwise manner using a pipette and incubated at room temperature for 30 minutes. Then, the mixed solution was added in a dropwise manner to a platE culture dish prepared by seeding separated platE cells using a pipette. Then, the cells were incubated for about 16 hours at 37°C in a 5% CO₂ cell incubator. After culturing, the culture medium was removed, washed twice with warm PBS, and 3 ml of DMEM medium without antibiotics added per 60 mm culture dish was dispensed and the cells were cultivated for additional 48 hours in a 37°C 5% CO₂ cell incubator. After the culture was completed, only the culture supernatant was transferred and filtered using a 0.45 um filter (ADVANTEC). The filtered supernatant containing the virus was immediately used for the experiment or was rapidly frozen using liquid nitrogen and then stored in a deep freezer at a temperature of -80°C.

### Isolation and activation of naive CD8 T Cells

Mice were sacrificed and spleens were removed. Then, the spleen in PBS was finely crushed, filtered through a mesh, transferred to a 15 ml centrifuge tube, and centrifuged for 5 minutes at 4°C and 1,500 rpm. The supernatant was removed, and the pellet was resuspended in 1 ml of Ack lysis buffer and incubated at room temperature for 3 minutes. Then, 10 ml of PBS was added and centrifuged for 5 minutes at 4°C and 1500 rpm. Fluorescently labeled anti-CD8 antibody and anti-CD44 antibody were mixed in PBS at a volume ratio of 100:1 to prepare an antibody mixture, centrifuged and added to the settled cell pellet, resuspended, and incubated at 4°C for 30 minutes. Then, 10 ml of PBS was added, followed by centrifugation for 5 minutes at 4°C and 1,500 rpm. After removing the supernatant, the pellet was resuspended in 1 ml of PBS, filtered through a cell strainer, and cell density was adjusted by adding an appropriate amount of PBS or RPMI medium (WELGENE) . Then, CD8⁺CD44^{low} cells (naive CD8 T cells) were isolated using a cell sorter (SH800, Sony Biotechnology, USA).

A 48-well plate (SPL Life Sciences) is coated with anti-CD3 antibody 1 hour 30 minutes to 2 hours before separation is complete. Specifically, PBS is added so that the concentration of anti-CD3 antibody (Biolegend) is 5 ug/ml. 150 pl of each well of a 48-well plate was dispensed. Thereafter, the antibody was coated by incubating for about 1 hour 30 minutes to 2 hours in a cell incubator at 37°C. The antibody mixture was removed from each well of the antibody-coated plate and washed with 150 pl of PBS, and the same washing process was repeated once more. After the separation was completed, the naive CD8 T cells were centrifuged at 4°C and 1,500 rpm for 5 minutes, the supernatant was removed, and the pellet was resuspended with RPMI medium supplemented with 10% FBS, streptomycin & penicillin (100 U/ml), 2-mercaptoethanol (Gibco). 1.5×10⁶ cells were added per well of the prepared anti-CD3 antibody-coated plate. At this time, anti-CD28 antibody (2 ug/ml, BD Pharmigen) and mIL-2 (100 U/ml, R&D Systems) were additionally treated. Then, it was cultured for 18-24 hours in a 37°C 5% CO₂ cell incubator.

### Transduction of Klf4 gene using retrovirus

The cells activated for 18 to 24 hours were transferred to a 15 ml centrifuge tube and centrifuged for 5 minutes at 25°C. at 1,500 rpm. Subsequently, Percoll solutions diluted to 30% and 60%, respectively, were prepared using 100% Percoll, RPMI medium, and PBS. The centrifuged pellet was resuspended in 4 ml of 30% Percoll solution, and then 3 ml of 60% Percoll solution was carefully placed on the bottom layer. Then, it was centrifuged for 20 minutes under the conditions of 25°C 2,000 rpm. At this time, acceleration and deceleration were set to minimum. After centrifugation, 3 ml of the upper layer was removed and the cells located at the interface were transferred to a new 15 ml centrifuge tube. Then, 10 ml of PBS was added and washed, followed by centrifugation at 25°C at 1,500 rpm for 5 minutes. After centrifugation, the pellet was washed once again with 10 ml of PBS, centrifuged for 5 minutes at 25°C and 1,500 rpm, and the cell pellet was resuspended in RPMI medium, and then the cells were counted. Then, cells were seeded at 5×10⁵ per well in 12-well uncoated plates (SPL Life Sciences).

After adding 1-2 ml of the retroviral supernatant (MigRI-Klf4 and MigRI) prepared above to each well, mIL-2 (100 U/ml) and polybrene (8 ug/ml, Sigma) were added. Then, the crevice was sealed with parafilm and centrifuged for 1 hour at 25°C at 1800 xg. In this case as well, acceleration and deceleration were set to minimum. After centrifugation, the parafilm was removed and incubated for 30 minutes in a 37°C 5% CO₂ cell incubator. Thereafter, the cells were transferred to a 15 ml centrifuge tube, washed by adding 10 ml of RPMI medium, and centrifuged at 25°C for 5 minutes at 1,500 rpm. After centrifugation, the cell pellet was resuspended in 10 ml of RPMI medium, washed once again, and centrifuged at 25°C at 1,500 rpm for 5 minutes. After centrifugation, the cell pellet was resuspended in PBS and immediately injected into mice intravenously to perform immune cell transfer (adoptive cell transfer), or to use for cell experiments through additional culture.

### Induction of Klf4 overexpression using APTO-253

In addition to transduction of the Klf4 gene using retrovirus, the present inventors predicted that the overexpression of the Klf4 gene endogenous in naive CD8 T cells would be possible by regulating the upstream signaling pathway of the transcription factor Klf4. Therefore, the present inventors hypothesize that overexpression of Klf4 protein by treating naive CD8 cells with APTO-253 (Cercek et al., Invest. New. Drug. 33(5): 1086-1092, 2015), which is known as a Klf4 expression inducer, would show an effect similar to transduction using a vector.

Accordingly, in the same manner as above, after separating naive CD8 T cells, naive CD8 T cells were seeded on a plate coated with anti-CD3 antibody and in the process of activation, anti-CD28 antibody (2 µg/ml) and mIL-2 (100 U/ml) was treated with 3 µM of APTO-253 (MedChemExpress, LLC). Then, it was cultured for 72 hours in a 37°C 5% CO₂ cell incubator. After completion of the culture, the cells were transferred to a 15 ml centrifuge tube, and the volume was adjusted to 3 ml by adding RPMI medium. After that, 3 ml of Histopaque (Sigma) was carefully placed in the lower layer. Then, centrifugation was carried out for 30 minutes at 25°C at 2,000 rpm, and at this time, acceleration and deceleration were set to a minimum. After centrifugation, the culture solution of the upper layer was removed, and the cells present at the interface were recovered, transferred to a 15 ml centrifuge tube, resuspended in 10 ml of PBS, and centrifuged for 5 minutes at 4°C and 1,500 rpm. After centrifugation, the supernatant was removed, the cell pellet was washed once more with 10 mL of PBS, and centrifuged for 5 minutes at 4°C and 1,500 rpm. After centrifugation, the supernatant was removed, the cell pellet was resuspended in an appropriate amount of PBS, and the cells were counted. Finally, 5×10⁵ cells were diluted in 200 pl of PBS and loaded into a 1 ml syringe, and then intravenously injected into mice for immune cell transplantation.

### Example 1: Study on the mechanism of exhaustion of CD8 T cells

As described above, when CD8 T cells are chronically exposed to cancer antigens, etc., exhaustion occurs, and thus exhausted CD8 T cells do not undergo normal secretion of active cytokines and cell division, and thus cannot perform a proper immune response and normal function do not recover even though time passes.

Accordingly, the present inventors performed an experiment to mimic the exhaustion situation *in vitro* in order to determine the cause of the phenomenon that CD8 T cells are activated and then become exhausted over time.

### 1-1: in vitro exhaustion experiment

To this end, the present inventors specifically isolated CD8 T cells from OT-I transgenic mice having OVA (ovalbumin)-peptide-specific T cell receptor (TCR) and repeatedly treated the OVA peptide for 5 days. All experimental sets were treated with IL-15 (5 ng/ml) and IL-7 (5 ng/ml) to increase the viability of CD8 T cells. After treatment with OVA peptide (10 ng/ml) and washing the next day, stimulation was repeatedly applied for 5 days in such a way that cytokines and OVA peptide were treated with the same composition again (repeated stimulation treatment group). At this time, as a control group, only a cytokine-treated group without OVA peptide treatment and a single stimulation treatment group in which OVA peptide (10 ng/ml) was treated for 2 days and washed, and then only cytokine was treated for 3days were added. In addition, a set treated with IL-21, which is known to help activate CD8 T cells, was also added (repetitive stimulation + IL-21 treated group) (FIG. 1A). After 5 days, live CD8 T cells were isolated using a cell sorter, RNA was extracted, reverse transcribed into cDNA, and the expression levels of various genes were confirmed by qRT-PCR. As a result, the expression of the Tox gene, a representative marker of exhaustion, was increased in the repeated stimulation treatment group that mimics the exhaustion state, but the expression of the Tox gene decreased in the repeated stimulation + IL-21 treatment group. When the expression pattern of Klf4 was determined in this situation, the expression of the Klf4 gene was increased in the repeated stimulation treatment group but it was confirmed that the expression of the Klf4 gene was significantly increased in the repeated stimulation + IL-21 treatment group (FIG. 1B), unlike to Tox gene. This shows that CD8 T cells respond to IL-21, an activator, resulting in higher expression of Klf4 gene, suggesting that Klf4 may be a factor related to the activation of CD8 T cells in a state of exhaustion.

### 1-2: in vivo exhaustion experiment

In order to confirm whether the results in the cell experiment of Example 1-1 can be reproduced in animal experiments, the present inventors performed exhaustion mimicry in vivo using experimental animals.

Specifically, spleen and tumor tissues were excised on day 18 after inoculation of MC38 cancer cells (3×10⁵) into C57BL/6 mice. Meanwhile, the animal experiments were performed according to the regulations of the Animal Experiment Ethics Committee of Seoul National University. In the spleen, CD44^{low} naive CD8 T cells with low CD44 expression and CD44^{high} effector CD8 T cells with high CD44 expression upon antigen stimulation were isolated using a cell sorter. In tumor tissues, based on information known from previous studies, Ly108⁺ CD8 T cells known as chronic progenitor cells, Ly108⁻CD69⁻Tim3⁺ CD8 T cells known as chronic effector cells, and finally Ly108⁻CD69⁺Tim3⁺ CD8 T cells, known as terminal exhausted CD8 T cells, were isolated using a cell sorter (FIG. 1C). RNA of the separated cells of 5 groups was extracted, reverse transcribed into cDNA, and expression levels of various genes were confirmed through qRT-PCR.

As a result, in the case of Klf4, it was confirmed that the overall expression level in CD8 T cells in the exhausted state (chronic progenitor cells, chronic effector cells, and terminal exhausted cells) present in the tumor tissue was higher than that of the naive CD8 T cells. In particular, it was confirmed that the expression of the Klf4 gene was highest in Ly108⁻CD69⁻Tim3⁺ CD8 T cells, known as chronic effector cells (FIG. 1D). From this, it was confirmed that CD8 T cells with high activity in the exhaustion state exhibited a higher Klf4 gene expression level in both *in vitro* and *in vivo* conditions.

### Example 2: Preparation of Klf4 overexpressing CD8 T cells through gene transduction

From the results of Example 1 above, the present inventors hypothesized that Klf4 gene can promote generation and function of chronic effector cells based on the discovery that Klf4 expression was higher in the effector cells that maintain anticancer activity in the exhaustion state.

Accordingly, in order to confirm that the above hypothesis is true, the present inventors attempted to determine whether the generation of effector cells is promoted, and exhaustion caused by chronic stimulation to the antigen is suppressed and function of the effector cells is enhanced when the expression of Klf4 in CD8 T cells is artificially increased.

To this end, first, the present inventors transduced the isolated CD8 T cells with the Klf4 gene to perform an experiment for overexpressing the Klf4 gene.

Specifically, after treating CD8 T cells with anti-CD3 antibody, anti-CD28 antibody, and mIL-2 for 24 hours, and then the CD8 T cells were transduced with a retrovirus MigRI vector (control), and a recombinant retroviral MigRI vector in which Klf4 gene is inserted (hereinafter referred to as 'Klf4') as an experimental group in order to overexpress Klf4 protein. Transduction using retrovirus was performed using the general method described above. Thereafter, CD8 T cells were rested by treatment with mIL-7 and mIL-15 for 3 days. Since the MigRI vector basically expresses GFP, the CD8 T cells transduced with MigRI vector were GFP⁺, and FACS analysis was performed. In the case of Klf4 GFP⁺ CD8 T cells, the proportion of the aforementioned chronic effector cell (Ly108⁻CD69⁻) subset was significantly higher than that of the non-transduced control group, MigRI GFP⁻, MigRI GFP⁺, and Klf4 GFP⁻, in which Klf4 was not overexpressed (FIG. 2A). This suggests that overexpression of Klf4 is an important factor in the development of chronic effector cell subsets.

Next, to determine whether Klf4 overexpression promotes the function of effector cells, CD8 T cells derived from PmelI mice having a T cell receptor that specifically recognizes the gp100 antigen were isolated and transduced with control retroviral vector (MigRI) and recombinant retroviral vector (Klf4) by the above-described method. After the gene was overexpressed, the target cancer cells overexpressing the gp100 antigen (MC38-gp100) were co-cultured for 6 hours to measure the ratio of dead target cancer cells. As a result, compared to the control group (MigRI), when cultured with PmelI mice-derived CD8 T cells overexpressing the Klf4 gene, the ratio of dead target cancer cells increased, indicating that Klf4 overexpressing CD8 T cells could more effectively kill cancer cells (FIG. 2B).

In addition, the *in vitro* exhaustion model performed in Example 1-1 was applied to confirm whether Klf4 overexpression promotes the function of chronic effector cells in the state of direct exhaustion. After 24 hours of treatment with OVA peptide, CD8 T cells were transduced with control retroviral vector (MigRI) and recombinant retroviral vector (Klf4), using retrovirus. After that, the OVA peptide was repeatedly treated for the remaining 4 days to induce *in vitro* exhaustion, and MigRI GFP⁺ and Klf4 GFP⁺ CD8 T cells were isolated using a cell sorter, and then gene expression was evaluated by qRT-PCR using the primer pairs shown in Table 1. As a result, Klf4 overexpressing CD8 T cells confirmed that Klf4 gene expression was much higher than that transduced with the control vector (MigRI), and at the same time, it was confirmed that the expression of the Tox gene, a marker of exhaustion, was significantly reduced (FIG. 2c). In addition, the amounts of granzyme B and interferon-gamma (IFN-γ) secreted from these CD8 T cells were measured through FACS analysis, and cytokine secretion was significantly increased in Klf4-overexpressed CD8 T cells (FIGs. 2D and 2E). From this result, it was confirmed that higher expression level of Klf4 gene promotes the formation of chronic effector cells (Ly108⁻CD69⁻) and the ability to kill target cancer cells, and suppresses further exhaustion in CD8 T cells in the exhaustion state as well as promotes secretion of more active cytokines.

**Table 1**

| | Primers used for qRT-PCR | | |
|---|---|---|---|
| Gene | Primers | sequences (5' → 3') | SEQ ID NOs |
| klf4 | forward primer | GTGCCCCGACTAACCGTTG | 7 |
| | reverse primer | GTCGTTGAACTCCTCGGTCT | 8 |
| tox | forward primer | CAACTCAAAGCCGTCAGTAT | 9 |
| | reverse primer | GCTGAGGAGTCATTCCTGGT | 10 |

### Example 3: Analysis of suppression of exhaustion state through animal experiments

### 3-1: Animal experiment through Klf4 gene transduction

From the above results, the present inventors used a mouse tumor model to determine whether overexpression of Klf4 in CD8 T cells could inhibit cancer development by increasing cell activity even *in vivo.* Specifically, 3×10⁵ gp100-expressing MC38 cancer cells (MC38-gp100) were inoculated into Rag2 KO mice, and a day later, and then CD8 T cells isolated from PmelI transgenic mice transduced which express gp100-specific TCR (Jackson Laboratory, received from the National Cancer Center) were transduced with the above-mentioned expression vector (MigRI and Klf4). 1×10⁶ transduced CD8 T cells were administered to the tumor model mice through intravenous injection. The tumor volume was checked until the 15^{th} day after inoculation of cancer cells, and mice were sacrificed at the end of the experiment and used for various analyses (FIG. 3A). Prior to transfer of transduced CD8 T cells into the mice, the expression level of Klf4 gene was confirmed. As a result, it was confirmed that the Klf4 gene was successfully overexpressed in the experimental group (FIG. 3B). In addition, it was confirmed that mice administered with CD8 T cells whose overexpression of Klf4 protein was induced showed dramatically decreased tumor volume compared to control mice administered with CD8 T cells transduced with MigRI (FIG. 3C). Further, FACS analysis shows that the expression level of Ki-67, as a measure of cell division, in the invasive CD8 T cell subsets present in the tumor tissue was significantly higher in CD8 T cells transduced with Klf4 compared to the control (MigRI) (FIG. 3D) . As well, it was confirmed that all of the active cytokines such as granzyme B, IFN-γ, TNF-α were increased (FIGs. 3E and 3F). From these results, it was confirmed that the CD8 T cells transduced with Klf4 gene showed much better anticancer immune response than the control group.

### 3-2: Analysis of suppression of exhaustion state using Klf4 inducers

From the results of Example 3-1, the present inventors hypothesized that if a drug capable of inducing Klf4 gene expression is used instead of transduction of the Klf4 gene, the anticancer effect could be increased according to the above results. An animal experiment was performed using APTO-253, which is known as an inducer of Klf4. Specifically, 3×10⁵ MC38 cancer cells were injected into Rag2 KO mice lacking lymphocytes, and the next day, 5×10⁵ CD8 T cells treated with PBS or APTO-253 for 3 days were intravenously administered. The tumor volume was checked until the 15^{th} day after inoculation of cancer cells, and mice were sacrificed at the end of the experiment and used for various analyses (FIG. 4A). As a result of checking the Klf4 gene expression level in CD8 T cells treated with APTO-253 for 3 days prior to transfer into the mice, CD8 T cells treated with APTO-253 showed increased Klf4 gene expression level compared to CD8 T cells treated with PBS (FIG. 4B). In addition, it was confirmed that mice administered with CD8 T cells treated with APTO-253 showed decreased tumor growth compared to the mice administered with PBS-treated CD8 T cells, being consistent with the results from the Klf4 overexpression experiment analysis (FIG. 4C). From these results, it was confirmed that, in addition to Klf4 gene transduction, the increase in Klf4 gene expression in CD8 T cells through the Klf4 inducer APTO-253 also enhances the anticancer immune response of CD8 T cells.

According to an embodiment of the present invention, it was proved that CD8 T cells overexpressing the Klf4 gene by gene transduction or drug treatment suppress the immune exhaustion effect induced by repeated antigen stimulation in the living body, thereby killing cancer cells effectively through an innate immune response.

### Example 4: Preparation of CAR-CD8 T cell whose expression is induced

### 4-1: Construction of EpCAM-Binding CAR construct

A schematic diagram showing an exemplary third-generation CAR construct is provided in FIG. 5. The CAR construct is produced as follows. The nucleotide sequence for the cDNA encoding a fusion protein CAR, comprising the amino acid sequences of anti-EpCAM scFv (SEQ ID NO: 11), CD8α hinge (SEQ ID NO: 12), CD28 TM (SEQ ID NO: 13), CD28 ICD (SEQ ID NO: 14), and CD3ζ ICD (SEQ ID NO: 15) linked in tandem (EpCAM-CD28-CD3ζ, FIG. 5) is synthesized by standard techniques, PCR-amplified, and ligated into pCLPS (Parry et al., J. Immunol., 171: 166-174, 2003), a third generation self-inactivating lentiviral vector based on pRRL-SIN-CMV-eGFP-WPRE (Dull et al., J. Virol. 72: 8463-8471, 1998), or pELNS (Carpenito et al., Proc. Natl. Acad. Sci. USA 106: 3360-3365, 2009), which differs from pCLPS by replacing CMV with EF-1α as the promoter for transgene expression. The encoded CAR comprises an scFv for binding to EpCAM (SEQ ID NO: 11).

### 4-2: Construction of anti-Trop-2 CAR construct

The lentiviral vector for expressing the CAR comprising anti-Trop-2 scFv (SEQ ID NO: 16), CD8α hinge, CD28, and CD3ζICD linked in tandem (anti-Trop-2-CD28-CD3ζ, FIG. 5) is constructed as described above except that the nucleotide sequence encoding anti-EpCAM-scFv is replaced by that of anti-Trop-2 scFv.

### 4-3: Construction of anti-CEACAM6 CAR construct

The lentiviral vector for expressing the CAR comprising anti-CEACAM6 scFv (SEQ ID NO: 17), CD8α hinge, CD28 TM, and CD3ζ ICD linked in tandem (anti-CEACM6-CD28-CD3ζ, FIG. 5) is constructed as described above except that the nucleotide sequence encoding anti-EpCAM is replaced by that of anti-CEACAM6 scFv.

### 4-4: Construction anti-CEACAM5 CAR construct

The lentiviral vector for expressing the CAR comprising anti-CEACAM5 scFv (SEQ ID NO: 18), CD8α hinge, CD28 TM, and CD3ζ ICD linked in tandem (anti-CEACAM5-CD28- CD3ζ, FIG. 5) is constructed as described above except that the nucleotide sequence encoding anti-EpCAM scFv is replaced by that of anti-CEACAM5 scFv.

### Example 5: Production of lentiviral particles including CAR constructs

High-titer, replication-defective lentiviral vectors constructed as described in the Examples above are produced and concentrated as described by Parry et al. (J. Immunol., 171: 166-174, 2003). Briefly, HEK 293T cells (ATCC CRL-3216) are cultured in RPMI 1640, 10% heat-inactivated FCS, 2 mM glutamine, 100 U/mL penicillin, and 100 ug/mL streptomycin sulfate. Cells are seeded at 5×106 per T 150 tissue culture flask 24 h before transfection with 7 µg of pMDG.1 (VSV-G envelop), 18 µg of pRSV.rev (HIV-1 Rev encoding plasmid), 18 µg of pMDLg/p.RRE (packaging plasmid), and 15 µg of the lentiviral vector of interest using Fugene 6 (Roche Molecular Biochemicals). Media are changed 6 h after transfection and the viral supernatant is harvested at 24 and 48 h posttransfection. Viral particles are concentrated 10-fold by ultracentrifugation for 3 h at 28,000 rpm with a Beckman SW28 rotor.

### Example 6: Transduction of T Cells with CAR lentiviruses

For certain purposes, T cells from normal individuals may be used with the subject CAR constructs for construct testing and design. Primary human CD4⁺ and CD8⁺ T cells are isolated from the PBMCs of healthy volunteer donors following leukapheresis by negative selection with RosetteSep kits (Stem Cell Technologies). T cells are cultured in complete media (RPMI 1640 supplemented with 10% heat-inactivated FCS, 2 mM glutamine, 100 U/mL penicillin, 100 ug/mL streptomycin sulfate, and 10 mM HEPES), stimulated with monoclonal anti-CD3 and anti-CD28 coated beads for 12 to 24 h, and transduced with a lentiviral vector of interest at MOI (multiplicity of infection) of 5 to 10. Human recombinant IL-2 is added every other day to a 50 U/mL final concentration and a cell density of 0.5 to 1.0×10⁶ cells/mL is maintained. The Klf4 construct may be incorporated into the CAR construct or the transduction of the Klf4 construct may be performed simultaneously with the transduction of a separate CAR construct or may be performed sequentially.

### Example 7: Generation and assessment of autologous CAR-T Cells from cancer patients

The method as described by Brentjens et al. (Sci. Transl. Med. 5: 177ra38, 2013) is followed. Briefly, PBMCs are obtained from cancer patients by leukapheresis, washed, and cryopreserved. T cells are isolated from thawed leukapheresis product, activated with Dynabeads Human T-Activator CD3/CD28 magnetic beads (Invitrogen), and transduced with a lentiviral vector of interest. Transduced T cells are further expanded with the WAVE bioreactor to achieve the desired modified T cell dose.

Modified T cells are assessed for persistence in patient peripheral blood and bone marrow by FACS, anti-tumor activity by in vitro killing of antigen-positive cancer cells, and cytokine profiles by analyzing serial serum samples obtained before and after infusion of modified T cells with the Luminex IS 100 System and commercially available 39-plex cytokine detection assays (Brentjens, R. L. et al., Blood 118: 4817-4823, 2011).

While the present invention has been described with reference examples and experimental examples, it is to be understood that the invention is not limited to the disclosed exemplary examples, and on skilled in the art may comprehend that there are various modifications and equivalent examples. Accordingly, the true scope of the present invention should be determined by the technical idea of the appended claims.

### INDUSTRIAL APPLICABILITY

The methods and substances according to an embodiment of the present invention may be used for the manufacture of medicines, particularly anticancer agents.

## Claims

1. An *in vitro* method of suppressing the exhaustion state of CD8 T cells comprising inducing overexpression of Klf4 protein in CD8 T cell selected from the group consisting of a) CD8 T cells, b) a cell population comprising the CD8 T cells, and c) transduced CAR-CD8 T cells prepared by transducing the CD8 T cells with a gene encoding a chimeric antigen receptor (CAR).

2. An *in vitro* method of proliferating CD8 T cells comprising inducing overexpression of Klf4 protein in CD8 T cells selected from the group consisting of a) CD8 T cells, b) a cell population comprising the CD8 T cells, and c) transduced CAR-CD8 T cells prepared by transducing the CD8 T cells with a gene encoding a chimeric antigen receptor (CAR).

3. An *in vitro* method of enhancing immune response of CD8 T cells comprising inducing overexpression of Klf4 protein in CD8 T cells selected from the group consisting of a) CD8 T cells, b) a cell population comprising the CD8 T cells, and c) transduced CAR-CD8 T cells prepared by transducing the CD8 T cells with a gene encoding a chimeric antigen receptor (CAR).

4. The method according to claim 1, the overexpression of Klf4 protein is performed by transfecting the CD8 T cell-containing cells with an expression vector containing a polynucleotide encoding the Klf4 protein; or treating the CD8 T cell-containing cells with a Klf4 inducer.

5. The method according to claim 4, wherein the expression vector is a viral vector or a non-viral vector.

6. The method according to claim 5, wherein the viral vector is an adeno-associated virus (AAV) vector, an adenovirus vector, an alphavirus vector, a herpes simplex virus vector, or a vaccinia virus vector, Sendaivirus vector, flavivirus vector, radovirus vector, retroviral vector, herpesvirus vector, poxvirus vector or lentiviral vector.

7. The method according to claim 5, wherein the non-viral vector is mRNA, a DNA vector, nanoparticles, cationic polymer, exosome, extracellular vesicle or liposome.

8. The method according to claim 7, wherein the mRNA is used as alone or in combination with a non-viral vector other than the mRNA.

9. The method according to claim 4, wherein the expression vector further comprises a polynucleotide encoding one or more immune-stimulating peptides.

10. The method according to claim 9, wherein the immune-stimulating peptide is CD28, ICOS (inducible costimulator), CTLA4 (cytotoxic T lymphocyte associated protein 4), PD1 (programmed cell death protein 1), BTLA (B and T lymphocyte associated protein), DR3 (death receptor 3), 4-1BB, CD2, CD40, CD40L, CD30, CD27, signaling lymphocyte activation molecule (SLAM), 2B4 (CD244), NKG2D (natural-killer group 2, member D)/DAP12 (DNAX-activating protein 12), TIM1 (T-cell immunoglobulin and mucin domain containing protein 1), TIM2, TIM3, TIGIT, CD226, CD160, LAG3 (lymphocyte activation gene 3), B7-1, B7-H1, GITR (glucocorticoid-induced TNFR family related protein), Flt3 ligand (fms-like tyrosine kinase 3 ligand), flagellin, herpesvirus entry mediator (HVEM), or the cytoplasmic domain of OX40L [ligand for CD134(OX40), CD252], or a linkage of two or more thereof.

11. The method according to claim 4, wherein the Klf4 inducer is APTO-253.

12. A pharmaceutical composition for treating cancer comprising CD8 T cells in which Klf4 protein is overexpressed as an effective ingredient.

13. The pharmaceutical composition according to claim 12, wherein the CD8 T cells are autologous cells isolated from a subject in need of treatment or heterologous cells isolated from other subject.

14. The pharmaceutical composition according to claim 12, wherein the CD8 T cells in which Klf4 protein is overexpressed are prepared by transducing CD8 T cells with a polynucleotide encoding Klf4 protein and/or treating the CD8 T cells with a Klf4 inducer.

15. A transformed CD8 T cell which is transformed to overexpress the Klf4 protein.

16. The transformed CD8 T cell according to claim 15, wherein the transduced CD8 T cell is prepared by transducing CD8 T cells or a cell population comprising the CD8 T cell with an expression vector containing a polynucleotide encoding a Klf4 protein.

17. The transformed CD8 T cell according to claim 16, wherein the expression vector is a viral vector or a non-viral vector.

18. The transformed CD8 T cell according to claim 17, wherein the viral vector is an adeno-associated virus (AAV) vector, an adenovirus vector, an alphavirus vector, a herpes simplex virus vector, a vaccinia virus vector, or a Sendai virus vector, a flavivirus vector, a radovirus vector, a retroviral vector, a herpesvirus vector, a poxvirus vector or a lentiviral vector.

19. The transformed CD8 T cell according to claim 17, wherein the non-viral vector is mRNA, a DNA vector, nanoparticles, cationic polymer, exosome, extracellular vesicle, or a liposome.

20. The transformed CD8 T cell according to claim 19, wherein the mRNA is used alone or in combination with a non-viral vector other than the mRNA.

21. A transformed CAR-CD8 T cell which is transduced to express heterologous Klf4 protein and a chimeric antigen receptor (CAR) .

22. The transformed CAR-CD8 T cell according to claim 21, wherein the CAR is a fusion protein comprising a single chain-based antibody mimetic, a transmembrane domain, a costimulatory domain and a cytoplasmic signaling domain.

23. The transformed CAR-CD8 T cell according to claim 22, wherein the single chain-based antibody mimetic binds to a cancer antigen or an antigen derived from a pathogen specifically.

24. The transformed CAR-CD8 T cell according to claim 23, wherein the cancer antigen is EpCAM (epithelial cell adhesion molecule), Trop-2 (trophoblast cell surface antigen 2), CEACAM5 (CEA cell adhesion molecule 5), CEACAM6 (CEA cell adhesion molecule 6), carcinoembryonic antigen (CEA), prostate-specific antigen prostatic acid phosphatase (PAP), prostate-specific membrane antigen (PSMA), Her2/neu, MUC-1, BCR/ABL, alpha-fetoprotein (AFP), an antigen derived from Epstein-Barr virus such as LMP2a, an antigen derived from human hepatitis B virus (HBV), human hepatitis C virus, Proteinase 3, WT-1, G250, melanoma antigen gene (MAGE), B melanoma antigen (BAGE), G melanoma antigen, NY-ESO-1, tyrosinase, tyrosinase-related protein-1 (TRP-1), TRP-2, gp100, MART-1, Ig Idiotype, CDK4, caspase-8, β-catenin, BCR/ABL, human papilloma virus antigen (HPV E6/E7), HHV-8, 5T4, p53, CA-125, CA-72-4, CA-15-3, or CA-19-9.

25. The transformed CAR-CD8 T cell according to claim 23, wherein the antigen derived from a pathogen is an antigen derived from a pathogenic microorganism, a virus or a parasite.

26. The transformed CAR-CD8 T cell according to claim 25, wherein the pathogenic microorganism is a pathogenic bacterium or a pathogenic fungus.

27. The transformed CAR-CD8 T cell according to claim 26, wherein the pathogenic bacterium is *Bordetella pertussis,* tetanus, diphtheria, *Helicobacter pylori, Pneumococcus* sp., *Mycobacterium tuberoculosis, Cholera* sp., *Staphylococcus* sp., *Shigella* sp., *Borrelia* sp. or *Salmonella* sp.

28. The transformed CAR-CD8 T cell according to claim 26, wherein the pathogenic fungus is *Candida* sp., *Trichophyton* sp., *Aspergillus* sp., *Fonsecaea* sp., *Epidermophyton* sp., *Piedraia* sp., *Malassezia* sp., *Pseudallescheria* sp., *Basidiobolus* sp., *Conidiobolus* sp., *Rhinosporidium* sp., *Paracoccidioides* sp., *Cryptococcus* sp., *Blastomyces* sp., *Sporothrix* sp., *Mucor* sp., *Absidia* sp., *Rhizopus* sp., *Pneumocystis* sp., *Wangiella* sp.,, *Phialophora* sp., or *Schizophyllum* sp.

29. The transformed CAR-CD8 T cell according to claim 25, wherein the virus is influenza virus, human papilloma virus (HPV), vesicular stomatitis virus, cytomegalovirus (CMV), hepatitis A virus (HAV), hepatitis B virus (HBV), hepatitis C virus (HCV), hepatitis D virus (HDV), hepatitis G virus (HGV), respiratory synctytial virus (RSV), herpes simplex virus (HSV), or human immunodeficiency virus (HIV).

30. The transformed CAR-CD8 T cell according to claim 22, wherein the single chain-based antibody mimetic is scFv, sdAb(single domain antibody), VHH, VNAR, Affibody, Affilin, Affimer, Affitin, Alphabody, Anticalin, Avimer, DARPin, Fynomer, Kunitz domain peptide, monobody, nanoCLAMP, variable lymphocyte receptor (VLR) or repebody.

31. The transformed CAR-CD8 T cell according to claim 22, wherein the transmembrane domain is a transmembrane domain derived from 4-1BB/CD137, activated NK cell receptor, immunoglobulin protein, B7-H3, BAFFR, BLAME(SLAMF8), BTLA, CD100(SEMA4D), CD103, CD160 (BY55), CD18, CD19, CD19a, CD2, CD247, CD27, CD276 (B7-H3), CD28, CD29, CD3 delta, CD3 epsilon, CD3 gamma, CD3 zeta, CD30, CD4, CD40, CD49a, CD49D, CD49f, CD69, CD7, CD84, CD8, CD8 alpha, CD8 beta, CD96 (Tactile), CDlla, CDllb, CDllc, CDlld, CDS, CEACAM1, CRT AM, cytokine receptor, DAP-10, DNAM1(CD226), Fc gamma receptor, GADS, GITR, HVEM (LIGHTR), IA4, ICAM-1, ICAM-1, Ig alpha (CD79a), IL-2R beta, IL-2R gamma, IL-7R alpha, inducible T-cell costimulatory (ICOS), integrin, ITGA4, ITGA4, ITGA6, ITGAD, ITGAE, ITGAL, ITGAM, ITGAX, ITGB2, ITGB7, ITGBl, KIRDS2, LAT, LFA-1, LFA-1, a ligand binding to CD83, LIGHT, LTBR, Ly9 (CD229), lymphocyte function-associated antigen-1 (LFA-1; CDl-la/CD18), MHC type 1 molecule, NKG2C, NKG2D, NKp30, NKp44, NKp46, NKp80 (KLRF1), OX-40, PAG/Cbp, programmed cell death-1 (PD-1), PSGL1, SELPLG (CD162), signaling lymphocytic activating molecule (SLAM), SLAMF1 (CD150 or IPO-3), SLAMF4 (CD244; 2B4), SLAMF6 (NTB-A; Lyl08), SLAMF7, SLP-76, TNF receptor protein, TNFR2, TNFSF14, toll ligand receptor, TRANCE/RANKL, VLA1, or VLA-6.

32. The transformed CAR-CD8 T cell according to claim 22, the costimulatory domain is a cytoplasmic domain or a conjugate of at least two or more among selected from the group consisting of CD28, ICOS (inducible costimulator), CTLA4 (cytotoxic T lymphocyte associated protein 4), PD1 (programmed cell death protein 1), BTLA(B and T lymphocyte associated protein), DR3(death receptor 3), 4-1BB, CD2, CD40, CD30, CD27, SLAM(signaling lymphocyte activation molecule), 2B4(CD244), NKG2D (natural-killer group 2, member D)/DAP12 (DNAX-activating protein 12), TIM1 (T-Cell immunoglobulin and mucin domain containing protein 1), TIM2, TIM3, TIGIT, CD226, CD160, LAG3 (lymphocyte activation gene 3), B7-1, B7-H1, GITR (glucocorticoid-induced TNFR family related protein), HVEM(herpesvirus entry mediator) and OX40L (CD252).

33. The transformed CAR-CD8 T cell according to claim 22, wherein the cytoplasmic signaling domain is one or more cytoplasmic domains selected from the group consisting of CD3ζ, CD28, CD27, OX40/CD134, 4-1BB/CD137, FcεRIγ, ICOS/CD278, IL-2Rβ/CD122, IL-2Rα/CD132, DAP10, DAP12, and CD40.

34. A composition comprising the transformed CD8 T cell of claim 14 or the transformed CAR-CD8 T cell of claim 21.

35. The composition according to claim 34, wherein the composition is used for treating a disease requiring an innate immune response.

36. The composition according to claim 35, wherein the disease requiring an innate immune response is cancer, a bacterial infection, a fungal infection, a viral infection, or a parasitic infection.
